(19) Europäisches Patentamt
European Patent Office
Office européen des brevets

(11) **EP 3 112 846 B1**

(12) **EUROPÄISCHE PATENTSCHRIFT**

(45) Veröffentlichungstag und Bekanntmachung des Hinweises auf die Patenterteilung:
**17.05.2017 Patentblatt 2017/20**

(51) Int Cl.:
**G01N 21/39** *(2006.01)*     **G01J 3/42** *(2006.01)*
**G01J 3/433** *(2006.01)*

(21) Anmeldenummer: **15174243.4**

(22) Anmeldetag: **29.06.2015**

(54) **VERFAHREN ZUR BESTIMMUNG DER KONZENTRATION EINER GASKOMPONENTE UND SPEKTROMETER DAFÜR**

METHOD OF DETERMINING THE CONCENTRATION OF A GAS COMPONENT AND SPECTROMETER FOR SAME

PROCEDE DE DETERMINATION DE LA CONCENTRATION D'UN COMPOSANT GAZEUX ET SPECTROMETRE ASSOCIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.01.2017 Patentblatt 2017/01**

(73) Patentinhaber: **SICK AG**
**79183 Waldkirch (DE)**

(72) Erfinder:
• **Beyer, Thomas**
**79111 Freiburg (DE)**

• **Edler, Julian**
**79312 Emmendingen (DE)**

(56) Entgegenhaltungen:
**DE-B3-102012 223 874**

• **KRAETSCHMER T ET AL: "Background-free absorption spectroscopy using delayed balanced detection", APPLIED PHYSICS B ; LASERS AND OPTICS, SPRINGER, BERLIN, DE, Bd. 98, Nr. 2-3, 11. Dezember 2009 (2009-12-11), Seiten 249-252, XP019779927, ISSN: 1432-0649**

**Beschreibung**

Die Erfindung betrifft ein Verfahren zur Bestimmung der Konzentration einer Gaskomponente und ein Spektrometer zur Durchführung des Verfahrens.

Zur Bestimmung der Konzentration einer Gaskomponente eines Messgases sind verschiedene Verfahren bekannt. Dazu gehören die direkte Absorptionsspektroskopie (DAS) und die Wellenlängenmodulationsspektroskopie (WMS).

In der direkten Absorptionsspektroskopie wird die Wellenlänge eines Lasers über eine Stromrampe verändert und das Detektorsignal aufgezeichnet. Durch das Messgas wird beim Durchlaufen der Stromrampe, wenn der Laser den Bereich der Absorptionslinie durchfährt, entsprechend des Lambert-Beer'schen Gesetzes Licht absorbiert.

$$I = I_0 * e^{-\alpha(\lambda)cL}$$

$\alpha(\lambda)$ ist die wellenlängenabhängige Absorption,
c ist die Gaskonzentration,
und L ist die Strecke, über die das Gas absorbiert.

Das Detektorsignal wird dadurch so verformt, dass im Vergleich zum Detektorsignal ohne Gasabsorption eine Absorptionslinie zu erkennen ist, die dann z.B. durch einen Fit mit einer physikalischen Modellfunktion (in der Regel eine Voigt-Absorptionslinie) angefittet wird und so die Absorptionsfläche (Fläche unter der Absorptionskurve) bestimmt werden kann, die proportional zur Gaskonzentration ist.

Die Wellenlängenmodulationsspektrometrie (WMS) ist eine Form von optischer Absorptionsspektroskopie, die eine Detektion von sehr kleinen optischen Absorptionen ermöglicht, weil die Absorptionsmessungen von kleinen Frequenzen (nahe DC), bei denen die Lichtquellen großes Rauschen aufweisen, zu hohen Frequenzen verschoben werden, bei denen das Schrotrauschen (shot-noise) der begrenzende Faktor ist. Diese Frequenzverschiebung kann die Messempfindlichkeit um drei bis fünf Größenordnungen verbessern.

Die WMS wird in der Regel mit kontinuierlich durchstimmbaren Lasern, wie Diodenlasern (TDL), durchgeführt. Dabei wird die Wellenlänge langsam über eine Absorptionslinie des Messgases durchgestimmt und zusätzlich mit einer demgegenüber hohen Modulationsfrequenz f, typischerweise sinusförmig, leicht moduliert. Wenn der so wellenlängenmodulierte Lichtstrahl sich durch die Messstrecke ausbreitet, ergibt sich aus der Intensitätsänderung des Lasers und durch die Absorption des Messgases eine Amplitudenmodulation des Lichts. Wenn das Licht dann im Lichtempfänger detektiert und ein Empfangssignal in Abhängigkeit der Zeit erzeugt wird, enthält das Empfangssignal AC-Komponenten bei der Modulationsfrequenz f und seinen Obertönen 2f, 3f, 4f, etc.. Für die Auswertung kann eine der AC-Komponenten ausgewählt werden und in einem phasenempfindlichen Verfahren, z. B. mit einem Lock-in-Verfahren, ausgewertet werden. Dieses Verfahren wird auch als Demodulation bezeichnet. Das bei einer Demodulation bei der Frequenz nf erhaltene Signal wird als nf-Signal bezeichnet (n=1,2,3,...). Das demodulierte Signal enthält damit Information bezüglich der optischen Absorption und der Intensität des Lichtstrahls. Über die so gemessene Absorption können Konzentrationen einer Gaskomponente des untersuchten Messgases bestimmt werden.

Eine detaillierte Theorie, die die WMS und die Beziehungen zwischen der Form der Absorptionslinie und der Form des demodulierten Signals beschreibt, ist in "Frequency-modulation spectroscopy for trace species detection: theory and comparison among experimental methods," Applied Optics 31, 707-717(1992) gegeben. Die Signalform, die bei der WMS erhalten wird, wenn man langsam über die Absorptionslinie fährt und gleichzeitig die Wellenlänge bei der Frequenz nf moduliert, entspricht qualitativ der n-ten Ableitung der Absorptionslinie, weswegen für die WMS auch die Bezeichnung derivative Absorptionsspektroskopie verwendet wird.

Aus der DE 102 38 356 A1 ist ein Verfahren bekannt, bei dem die beiden Messverfahren WMS und DAS abwechselnd in aufeinanderfolgenden Perioden, angewendet und die detektierten Signale ebenfalls wechselweise zwei getrennten Mittelwertbildungen zugeführt ausgewertet werden. Bei der WMS-Auswertung können die Ergebnisse der DAS-Auswertung herangezogen werden, z. B. zur Kalibrierung. Damit erhält man die Kalibrationsfreiheit der direkten Absorptionsspektroskopie und die Genauigkeit der Wellenlängenmodulationsspektroskopie.

Bei einem aus der US 7,616,316 B2 bekannten Verfahren wird zwischen der DAS bei hohen und WMS bei niedrigen Konzentrationen der zu messenden Gaskomponente umgeschaltet. Es kommt also jeweils das am geeignetsten erscheinende Messverfahren zur Anwendung.

Aus der DE 10 2012 223 874 B3 werden beide Messverfahren, WMS und DAS, gleichzeitig oder wie in der DE 102 38 356 A1 abwechselnd angewendet und die beiden Messergebnisse durch Mittelwertbildung verknüpft, wodurch kleinere Fehler im Ergebnis erzielt werden können.

Kraetschmer et al "Background-free absorption spectroscopy using delayed balanced detection", Applied Physics B; Lasers And Optics, Springer, Berlin, DE, Bd.98, Nr. 2-3, 11. Dezember 2009, Seiten 249-252, ISSN: 1432-0649, beschreibt eine Spektroskopiemethode, die die Nachteile der WMS und der DAS vermeidet aber deren Vorteile nutzt und daher WMS und DAS ersetzen soll. Dies wird dadurch erreicht, dass die Laserdiode mit einer einfachen Rampe angesteuert wird (wie bei DAS) aber kein direktes Spektrum aufgenommen wird, sondern das Licht über zwei unterschiedlich lange optische Verzögerungsstrecken auf einen "balanced photoreceiver" fällt. Dieser gibt nur ein Differenzsignal der beiden Eingänge aus und damit eine dem 1f Signal einer WMS äquivalentes Signal.

Die Erfindung hat das Ziel, die funktionale Sicherheit (SIL; Safety-Integrated-Level) bei Gaskonzentrationsbestimmungen zu erhöhen, um Sicherheitsnormen, die SIL definieren, erfüllen zu können. In der funktionalen Sicherheit kann es sein, dass eine Fehlertoleranz von 1 gefordert wird. Dies bezieht sich normalerweise auf die Hardware. Unabhängig von der eingesetzten Hardware können bei der Gaskonzentrationsmessung aber wegen externer Einflüsse, wie Intensitätsfluktuationen, Druckänderungen, Druck, Temperatur und Interferenzen, nicht-zufällige Fehler auftreten. Es ist deshalb Aufgabe der Erfindung, ein im Hinblick auf die funktionale Sicherheit verbessertes Verfahren und Spektrometer zur Bestimmung einer Gaskomponente bereit zu stellen.

Eine solche Erhöhung der Sicherheit könnte durch Anwendung von zwei verschiedenen Messverfahren, DAS und WMS, wie durch die DE 102 38 356 A1 und die DE 10 2012 223 874 B3 bekannt, erzielt werden. Nachteilig ist aber, dass dies eine Verdoppelung des Aufwands bzgl. Zeit und Apparatur bedeutet. Bei der in der DE 10 2012 223 874 B3 offenbarten, gleichzeitigen Anwendung der Messverfahren muss der Laser mit einer Rampe plus Sinusmodulation angesteuert werden. Das ist für die DAS nachteilig, denn das direkte Detektorsignal bei der WMS ist nicht für eine Auswertung nach der DAS geeignet, da die Sinusmodulation nicht nur zu einer Leistungsmodulation führt, sondern auch zu einer Wellenlängenmodulation. Auch nach einer Mittelung über z.B. eine Sinusperiode ist die Absorption durch die Sinusmodulation verschliffen und die Auswertung schwierig und nicht mehr über einen Fit mit einem physikalischen Modell zu erreichen. Das Ergebnis aus einer solchen DAS ist also nicht so aussagekräftig, da der Laser dafür ungünstig angesteuert wird.

Die Erfindung löst deshalb das Problem anders und zwar durch ein Verfahren nach Anspruch 1 bzw. ein Spektrometer nach Anspruch 10.

Das erfindungsgemäße Verfahren zur Bestimmung der Konzentration einer Gaskomponente umfasst folgende Schritte:

- Erzeugen eines Lichtstrahls mit einer in einem Wellenlängenbereich variablen Wellenlänge,
- Führen des Lichtstrahls durch ein Messvolumen, in dem die zu bestimmende Gaskomponente vorliegt, wobei die Gaskomponente in dem Wellenlängenbereich eine Absorption aufweist,
- Durchstimmen des Wellenlängenbereichs,
- Detektieren der Intensität des Lichtstrahls nach Durchtritt durch das Messvolumen,
- während des Durchstimmens Speichern von Messpunkten, die jeweils aus einem Zeitpunkt und dazugehörigem Intensitätswert bestehen, wodurch eine direkte Absorptionslinie erhalten ist,
- Erzeugen einer künstlichen Messkurve aus den gespeicherten Messpunkten, durch Verschieben der Messpunkte auf der Zeitachse,
- wobei die Verschiebung derart erfolgt, dass sich nach Verschiebung der Messpunkte in dem Wellenlänge-Zeit-Verlauf eine künstliche Modulation ergibt,
- Auswerten der künstlichen Messkurve nach den Methoden der Wellenlängenmodulationsspektroskopie und Ermitteln eines ersten Konzentrationswertes daraus.

Die wesentliche Idee ist somit die Durchführung nur einer Messung und zwar mit einer Ansteuerung der Lasers, also einer Durchstimmung des Wellenlängenbereichs, für eine Messung nach der DAS. Die erhaltenen Messsignale aber werden dann nach den beiden Methoden der DAS bzw. WMS ausgewertet. Auf diese Weise werden auf zwei verschiedenen Wegen zwei Ergebnisse erhalten. Das ist nur möglich durch die Speicherung der Messpunkte und neue Aneinanderreihung (Verschiebung) der Messpunkte zur Erzeugung einer künstlichen Messkurve aber aus den bisherigen Messwerten, die dann nach der WMS ausgewertet werden können. Darin steckt die wesentliche Idee.

Der wesentliche Vorteil eines solchen Verfahrens ist, dass die Auswertung eines Detektorsignals mit zwei unterschiedlichen Auswertemethoden möglich ist, so dass externe Einflüsse keine nicht-zufälligen Fehler erzeugen können, wodurch ein hohes SIL Level erreicht werden kann.

Ein weiterer Vorteil ist, dass das "Hochfrequenzverhalten" des Lasers auf die WMS, d.h. Veränderungen des Lasers, die nur durch die schnelle Sinusmodulation des Laserstroms in der Laserwellenlänge auftreten, keine Rolle mehr spielen. Solcherlei Veränderungen ergeben sich aus der Tatsache, dass die Wellenlängenänderung vor allem durch thermische Effekte ausgelöst wird und diese normalerweise langsam vonstatten gehen und den schnellen Sinusmodulationen nicht folgen können, so dass ungewollt die Wellenlängenmodulation immer kleiner wird und nur die Intensitätsmodulation bestehen bleibt. Diese Veränderungen der Lasereigenschaften spielen mit dem erfindungsgemäßen Verfahren keine Rolle mehr, da die Messkurve für die WMS-Auswertung lediglich durch Umsortieren (Verschieben) der Messpunkte auf der Zeitachse erfolgt ist und die Messpunkte ohne die schnellen Sinusmodulationen erhalten wurden.

Ein weiterer Vorteil ist, dass die gesamte Messung sehr schnell ist, da nur noch die Abstimmung des Lasers entlang der Rampe (Durchstimmung des Wellenlängenbereichs) benötigt wird und keine Komponente mehr einer schnellen Sinusmodulation folgen muss. Höhere Repetitionsraten der Rampe (Durchstimmung) sind somit möglich, da die nachträglich durch Neusortierung der Messpunkte erzeugte Modulation, unabhängig ist von dem Frequenzgang der Verstärker für das Detektorsignal und keine Sinusmodulation aufgezeichnet werden muss.

Um ein bestimmtes SIL Level zu erreichen ist es sinnvoll, einen zweiten Konzentrationswert zu erhalten, was in Weiterbildung der Erfindung dadurch erfolgt, dass nach dem Speichern der Messpunkte eine Auswertung der direkten Absorptionslinie nach der Methode der direkten Absorptionsspektroskopie durchgeführt wird und ein zweiter Konzentrationswert bestimmt wird.

Damit können die beiden erzeugten Konzentrationswerte dazu verwendet werden, um durch eine Plausibilisierung der Werte zueinander eine höheres Level bzgl. Funktionaler Sicherheit zu erreichen.

Die beiden, auf den verschiedenen Wegen erhaltenen Konzentrationswerte können miteinander verglichen werden, um so einen der beiden Werte lediglich zum Verifizieren des anderen zu verwenden.

Zusätzlich oder alternativ könnte man aus den beiden Konzentrationswerten einen gemeinsamen Konzentrationswert durch Mittelwertbildung erzeugen, der dann einen kleineren Fehler als ein Einzelwert hat.

Es ist sinnvoll und reduziert den Aufwand, wenn das Durchstimmen des Wellenlängenbereichs ohne zusätzliche hochfrequente Modulation der Wellenlänge während des Durchstimmens erfolgt, so dass sich ein monotoner Wellenlänge-Zeit-Verlauf ohne hochfrequente Modulation ergibt.

In verschiedenen Weiterbildungen der Erfindung kann die künstliche Modulation einer gewünschten WMS Auswertung angepasst werden. Eine solche Anpassung kann sich auf die Modulation selbst beziehen, die typischerweise sinusförmig ist, aber auch rechteck- oder dreieckförmig sein kann. Auch können Amplitude und/oder Frequenz der künstlichen Modulation durch entsprechende Verschiebung der Messpunkte, für die Auswertung nach der Wellenlängenmodulationsspektroskopie angepasst ausgestaltet werden. Ebenso kann die Phase der künstlichen Modulation derart angepasst werden, dass bei der Auswertung nach der Wellenlängenmodulationsspektroskopie das Ergebnis nur im Realteil der Fourriertransformation steckt.

Solche Anpassungen der künstlichen Modulation, die sehr einfach zu bewerkstelligen sind, denn es müssen dazu nur die Messpunkte entsprechend verschoben, also anders und neu aneinandergereiht, werden, können die Auswertung in einer gewünschten Weise vereinfachen oder verbessern.

Ein erfindungsgemäßes Spektrometer, mit dem das Verfahren durchführbar ist, weist auf:

- eine Lichtquelle zum Erzeugen eines Lichtstrahls mit einer in einem Wellenlängenbereich variablen Wellenlänge,
- einem Messvolumen, in dem die zu bestimmende Gaskomponente vorliegt und durch das der Lichtstrahl verläuft,
- Ansteuermittel für die Lichtquelle zum Durchstimmen des Wellenlängenbereichs ohne zusätzliche hochfrequente Modulation der Wellenlänge während des Durchstimmens, so dass sich ein monotoner Wellenlänge-Zeit-Verlauf ohne hochfrequente Modulation ergibt,
- einen Lichtdetektor zum Detektieren der Intensität des Lichtstrahls nach Durchtritt durch das Messvolumen,
- Speichermittel zum Speichern von Messpunkten, die jeweils aus einem Zeitpunkt und dazugehörigem Intensitätswert bestehen, während des Durchstimmens, wodurch eine direkte Absorptionslinie erhalten ist,
- eine Auswerteeinheit zum Erzeugen einer künstlichen Messkurve aus den gespeicherten Messpunkten, durch Verschieben der Messpunkte auf der Zeitachse, wobei die Verschiebung derart erfolgt, dass sich nach Verschiebung und somit neuer Aneinanderreihung in dem Wellenlänge-Zeit-Verlauf eine zusätzliche hochfrequente Modulation ergibt,
- und zum Auswerten der künstlichen Messkurve nach den Methoden der Wellenlängenmodulationsspektroskopie und Ermitteln eines ersten Konzentrationswertes daraus.

Vorteilhafterweise ist die Auswerteeinheit derart ausgestaltet, dass die Messpunkte, die jeweils aus einem Zeitpunkt und dazugehörigem Intensitätswert bestehen, nach der Methode der direkten Absorptionsspektroskopie ausgewertet werden können und so ein zweiter Konzentrationswert bestimmbar ist.

In Weiterbildung ist Lichtquelle ein durchstimmbarer Laser, dessen Emissionswellenlänge durch einen Steuerstrom oder eine Steuerspannung veränderbar ist.

In Weiterbildung der Erfindung erfolgt das Durchstimmen durch lineare Änderung des Steuerstroms oder der Steuerspannung. Eine lineare Änderung ist einfach zu bewerkstelligen und erlaubt auch eine recht einfache Umsortierung (Verschiebung) der gespeicherten Messpunkte, um die zusätzliche Modulation einzubringen.

Alternativ könnte das Durchstimmen auch durch nicht lineare Änderung des Steuerstroms erfolgen. So könnte man beispielsweise den Laser mit einer beliebigen Pulsform ansteuern, was die Ansteuerung erheblich vereinfachen würde, womöglich aber auf Kosten eines größeren Aufwandes bei der Umsortierung der Messpunkte zur Erzielung der zusätzlichen Modulation.

Im Folgenden wird die Erfindung anhand eines Ausführungsbeispiels unter Bezugnahme auf die Zeichnung im Einzelnen erläutert. In der Zeichnung zeigen:

Fig. 1   eine schematische Darstellung des erfindungsgemäßen Spektrometers;

Fig. 2   eine qualitative, schematische Darstellung der Laseransteuerung;

Fig. 3   eine qualitative, schematische Darstellung eines Absorptionssignals einer Messgaskomponente;

Fig. 4     eine Darstellung wie Fig. 2 nach Verschieben der Punkte auf der Zeitskala;

Fig. 5     eine Darstellung eines Ausschnitts aus Fig. 4 zur Verdeutlichung der Verschiebung der Punkte auf der Zeitskala;

Fig. 6     eine Darstellung der künstlichen Messkurve nach Verschieben der Punkte auf der Zeitskala.

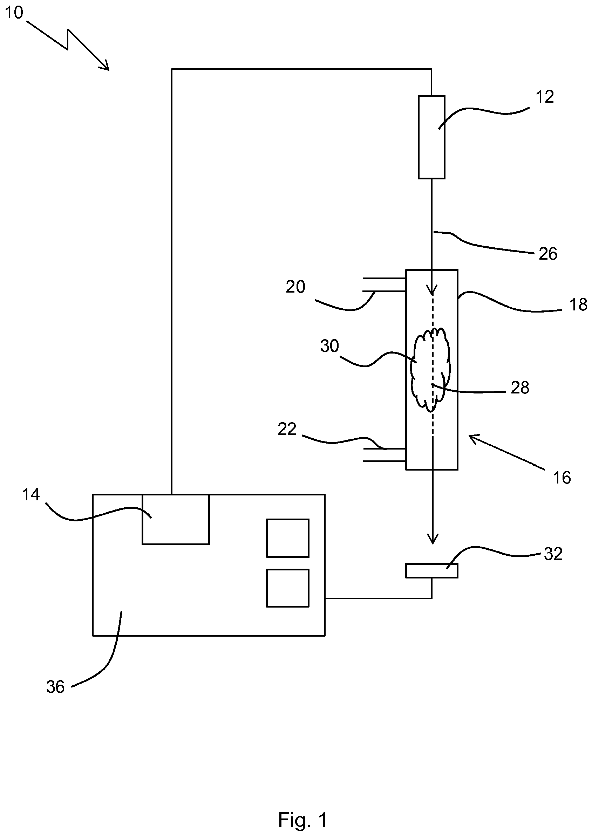

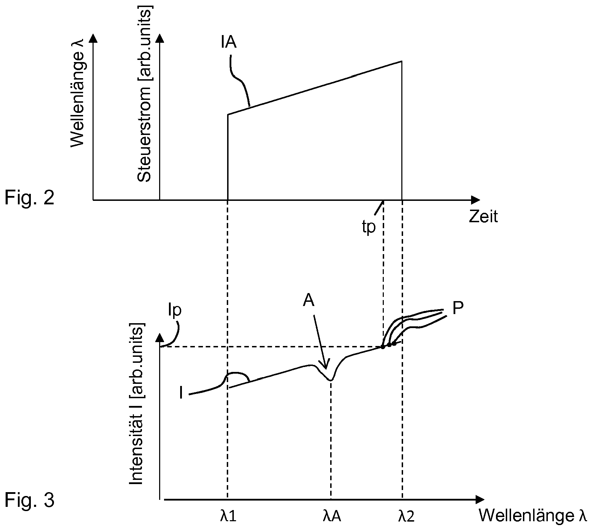

Ein in Fig. 1 schematisch dargestelltes, erfindungsgemäßes Spektrometer 10 weist eine Lichtquelle 12 auf, die vorzugsweise als durchstimmbarer Diodenlaser (tunable diode laser, TDL) ausgebildet ist, der mit Ansteuermitteln 14 ansteuerbar ist. Der Durchstimmbereich entspricht einem Wellenlängenbereich [$\lambda1$, $\lambda2$]. Zur Durchstimmung wird durch die Ansteuermittel 14 ein Steuerstrom IA an den Diodenlaser 12 gegeben, so dass in Abhängigkeit der Stromstärke eine entsprechende Wellenlänge $\lambda$ erzeugt wird. In Fig. 2 ist die Stromstärke IA des Steuerstroms in Abhängigkeit der Zeit t aufgetragen. Wenn sich die Stromstärke IA ändert, ändert sich auch die Wellenlänge $\lambda$, wie durch die zwei Ordinatenachsen in Fig. 2 und die Übereinanderdarstellung von Fig. 2 und 3 angedeutet werden soll.

Weiter weist das Spektrometer 10 ein Messvolumen 16 auf, das aus einer Messzelle 18 mit Messgaseinlass 20 und Messgasauslass 22 gebildet sein kann. Andere Anordnungen, z. B. offene Systeme ("open path") oder an einer Rohrleitung, die das Messgas führt ("cross duct"), angeschlossene Systeme sind denkbar. In der Messzelle 18 liegt ein Messgas 30 vor mit einer Gaskomponente, deren Konzentration gemessen werden soll.

Das Licht des Lasers 12 wird in die Messzelle 18 eingekoppelt. Der optische Weg innerhalb der Messzelle bildet eine optische Messstrecke 28. Der optische Weg kann über einen oder mehrere Reflektoren innerhalb oder außerhalb der Messzelle verlängert werden, zum Beispiel in Form einer White- oder Herriott Zelle, um so eine längere optische Messstrecke 28 zu erhalten.

In der Messzelle 18 liegt das Messgas 30 vor, das die zu messende Gaskomponente aufweist. Die Gaskomponente hat in dem durchstimmbaren Wellenlängenbereich eine Absorption A, so dass das die Messstrecke 28 durchlaufende Licht des Lasers 12 bei der Absorptionswellenlänge $\lambda A$ absorbiert wird. Dies ist in Fig. 3 dargestellt, die die durch die Messzelle 18 transmittierte Lichtintensität I in Abhängigkeit der Wellenlänge $\lambda$ zeigt.

Weiter ist ein Lichtdetektor 32 vorgesehen, der das Licht, das die Messstrecke durchlaufen hat, detektiert. Der Detektor 32 kann eine Photodiode, eine Lawinenphotodiode (Avalanche-Photo-Diode) oder auch ein Photomultiplier (PM) sein. Der Lichtdetektor 32 erzeugt in Abhängigkeit der Intensität des einfallenden Lichts ein Empfangssignal.

Das eine elektrische Empfangssignal enthält dann alle Informationen. Es wird optional verstärkt und/oder gefiltert und der Auswerteeinheit 36 zugeführt. Aus dem einen Empfangssignal werden in der Auswerteinheit 36 letztendlich die Konzentrationen der Gaskomponente ermittelt.

Die Bedeutung der einzelnen Komponenten, deren besondere Ausgestaltungen und Funktionen werden in der folgenden Beschreibung deutlich, wenn die Funktionsweise des erfindungsgemäßen Spektrometers 10 beschrieben wird. Dabei wird davon ausgegangen, dass die Funktionsweisen der DAS und der WMS, wie sie auch eingangs kurz erläutert wurden, prinzipiell bekannt sind.

Nach der Erfindung wird der durchstimmbare Laser 12 mittels der Ansteuermittel 38 angesteuert. Die Ansteuerung erfolgt bei Diodenlasern in der Regel über den Steuerstrom IA. Entsprechend dem Steuerstrom IA emittiert der Laser eine bestimmte Wellenlänge $\lambda$. Der so angesteuerte Laser 12 deckt den Wellenlängenbereich [$\lambda1$, $\lambda2$] ab (Fig. 2 und 3).

Die Ansteuerung des Lasers 12 erfolgt mit einer Stromrampe, wie sie die Fig. 2 zeigt. In diesem Beispiel ist die Stromrampe linear, d.h. der Steuerstrom IA ändert sich linear mit der Zeit. Zur Messung wird die Stromrampe mit einer Repetitionsrate wiederholt durchlaufen und so die Messung mit der Repetitionsfrequenz wiederholt.

Während des Durchstimmens des Wellenlängenbereichs werden Messpunkte P aufgenommen, die zusammen den Intensitätsverlauf I bilden, und von denen in Fig. 3 nur drei beispielhaft dargestellt sind. Jeder Messpunkt P besteht aus einem Zeitpunkt tp und dazugehörigem Intensitätswert Ip.

Der Intensitätsverlauf I zeigt die direkte Absorptionslinie A bei der Absorptionswellenlänge $\lambda A$. Die direkte Absorptionslinie A wird in der Auswerteeinheit 36 nach den Methoden der direkten Absorptionsspektroskopie ausgewertet und liefert einen zweiten Konzentrationswert.

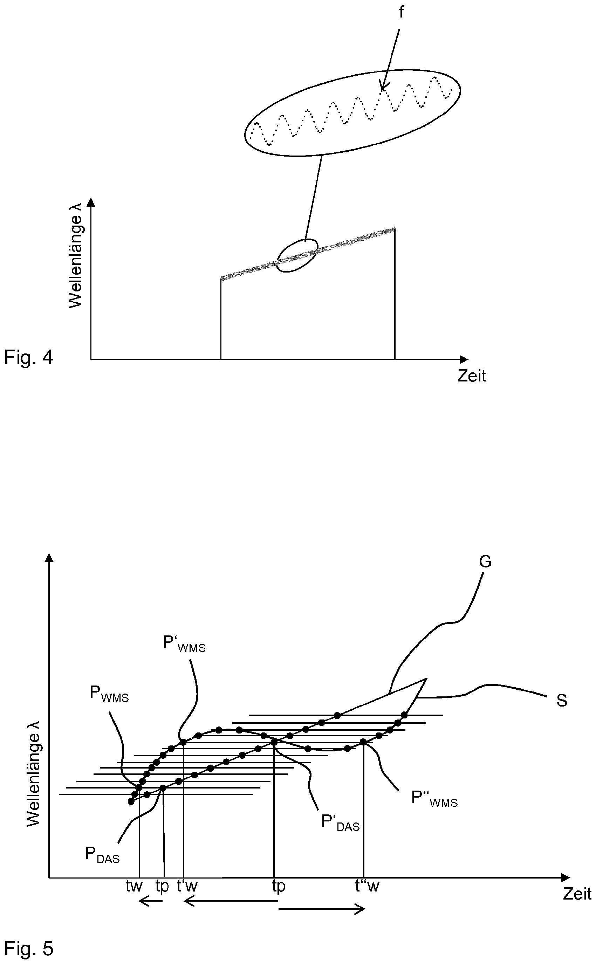

In einem nächsten Schritt werden nun die gespeicherten Messpunkte P einer Durchstimmung genommen und aus diesen ein neuer künstlicher Intensitätsverlauf, also eine künstliche Messkurve erzeugt. Dabei wird wie folgt vorgegangen. Und zwar werden die Messpunkte P auf der Zeitachse (Abszisse) verschoben und quasi neu aneinandergereiht oder anders ausgedrückt "umsortiert". Dabei erfolgt die Verschiebung derart, dass sich in einem nach Verschiebung (Neuaneinanderreihung) ergebenden Wellenlänge-Zeit Diagramm, wie in Fig. 4 dargestellt, eine zusätzliche hochfrequente Modulation f mit kleiner Amplitude zeigen würde und nicht ein linearer Verlauf, wie in Fig. 2. Die kleine Amplitude und hohe Frequenz ist in Fig. 4 nur in dem vergrößerten Ausschnitt zu erkennen.

Die Verschiebung (Umsortierung bzw. Neuaneinanderreihung) soll anhand der Fig. 5 erläutert werden. Dort ist nur ein kleiner Ausschnitt aus dem Wellenlänge-Zeit Diagramm der Fig. 4 dargestellt. Die in Fig. 4 eingezeichneten Punkte $P_{DAS}$ bzw. $P'_{DAS}$ sind über die Zeitpunkte tp den gespeicherten Messpunkten P bijektiv zugeordnet. Jeder Punkt $P_{DAS}$, $P'_{DAS}$ hat somit eine Zeitkoordinate tp und eine Wellenlängenkoordinate. Bei linearer Durchstimmung liegen die Punkte $P_{DAS}$ bzw. $P'_{DAS}$ auf einer Geraden G, wie das Fig. 5 zeigt.

Nun erfolgt aber die Verschiebung (Neuaneinanderreihung) und zwar so, dass danach sich der auf die Gerade G aufmodulierte sinusförmige Verlauf S ergibt. Dazu bedarf es einer Verschiebung des Punktes $P_{DAS}$ auf der Zeitachse (horizontal) zu einem neuen Zeitpunkt tw, so dass der Punkt $P_{DAS}$ zu $P_{WMS}$ verschoben ist. Dies erfolgt für die anderen Punkte analog. Um einen sauberen sinusförmigen Verlauf zu erhalten, müssen manche der Punkte $P_{DAS}$ mehrmals zu verschiedenen neuen Zeitpunkten verschoben werden. So muss z.B. der Punkt $P'_{DAS}$ von seinem Zeitpunkt tp einerseits zu t'w und andererseits zu t"w verschoben werden. Der Punkt $P'_{DAS}$ hat sich also quasi verdoppelt und taucht nach Neuaneinanderreihung zweimal auf. Je nach Frequenz und Amplitude des zu erreichenden sinusförmigen Verlaufs S und Steilheit der Geraden G kann es auch vorkommen, dass Messpunkte $P_{DAS}$ mehr als zweimal verschoben werden.

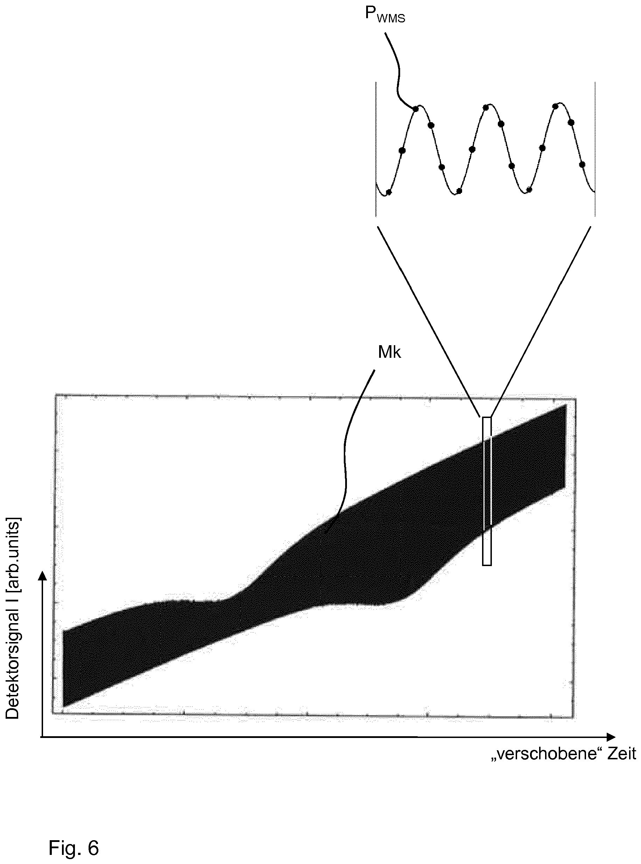

Mit den so verschobenen und neu "aufgereihten" Messpunkten P, die ja auch einen jeweiligen Intensitätswert beinhalten, wird nun eine künstliche Messkurve Mk, wie sie in Fig. 6 dargestellt ist, in der Auswerteeinheit konstruiert. Die Messkurve Mk der Fig. 6 setzt sich aus vielen einzelnen Messpunkten $P_{WMS}$ zusammen, die durch Verschieben auf der Zeitachse entsprechend den vorhergehenden Erläuterungen erzeugt sind und die wegen der hohen Frequenz der Modulation nicht einzeln erkennbar sind, sondern in einer solchen Darstellung, die den gesamten Durchstimmbereich darstellt (damit man die Absorption sieht), nur als "schwarze Masse" erscheinen.

Die künstliche Absorptionskurve Mk wird nun nach den Methoden der Wellenlängenmodulationsspektroskopie in der Auswerteeinheit 36 ausgewertet und daraus ein erster Konzentrationswert ermittelt.

## Patentansprüche

1. Verfahren zur Bestimmung der Konzentration einer Gaskomponente umfassend:

   - Erzeugen eines Lichtstrahls mit einer in einem Wellenlängenbereich ([$\lambda 1$, $\lambda 2$]) variablen Wellenlänge,
   - Führen des Lichtstrahls durch ein Messvolumen, in dem die zu bestimmende Gaskomponente vorliegt, wobei die Gaskomponente in dem Wellenlängenbereich eine Absorption aufweist,
   - Durchstimmen des Wellenlängenbereichs,
   - Detektieren der Intensität des Lichtstrahls nach Durchtritt durch das Messvolumen
   - während des Durchstimmens Speichern von Messpunkten, die jeweils aus einem Zeitpunkt und dazugehörigem Intensitätswert bestehen, wodurch eine direkte Absorptionslinie erhalten ist,
   - Erzeugen einer künstlichen Messkurve aus den gespeicherten Messpunkten, durch Verschieben der Messpunkte auf der Zeitachse,
   - wobei die Verschiebung derart erfolgt, dass sich nach Verschiebung in dem Wellenlänge-Zeit-Verlauf eine künstliche Modulation der Wellenlänge ergibt,
   - Auswerten der künstlichen Messkurve nach den Methoden der Wellenlängenmodulationsspektroskopie und Ermitteln eines ersten Konzentrationswertes daraus.

2. Verfahren nach Anspruch 1, **dadurch gekennzeichnet, dass** nach dem Speichern der Messpunkte eine Auswertung der direkten Absorptionslinie nach der Methode der direkten Absorptionsspektroskopie durchgeführt wird und ein zweiter Konzentrationswert bestimmt wird.

3. Verfahren nach Anspruch 2, **dadurch gekennzeichnet, dass** die beiden erzeugten Konzentrationswerte dazu verwendet werden, um durch eine Plausibilisierung der Werte zueinander eine erhöhte Sicherheit bzgl. Funktionaler Sicherheit zu erreichen.

4. Verfahren nach Anspruch 2 oder 3, **dadurch gekennzeichnet, dass** aus den beiden Konzentrationswerten ein gemeinsamer Konzentrationswert erzeugt wird.

5. Verfahren nach einem der vorhergehenden Ansprüche, **dadurch gekennzeichnet, dass** das Durchstimmen des Wellenlängenbereichs ohne zusätzliche hochfrequente Modulation der Wellenlänge während des Durchstimmens erfolgt, so dass sich ein monotoner Wellenlänge-Zeit-Verlauf ohne hochfrequente Modulation ergibt.

6. Verfahren nach einem der vorhergehenden Ansprüche, **dadurch gekennzeichnet, dass** das Durchstimmen des Wellenlängenbereichs in positiver Richtung mit einer nicht zwingend konstanten Geschwindigkeit >=0 und anschließender Änderung in negativer Richtung mit einer nicht zwingend konstanten Geschwindigkeit >=0 verändert wird. Dabei können beide Richtungen oder nur eine Richtung für die Auswertung herangezogen werden.

7. Verfahren nach einem der vorhergehenden Ansprüche, **dadurch gekennzeichnet, dass** die künstliche Modulation sinusförmig, rechteck- oder dreieckförmig ist.

8. Verfahren nach einem der vorhergehenden Ansprüche, **dadurch gekennzeichnet, dass** die Amplitude und/oder die Frequenz der künstlichen Modulation für die Auswertung nach der Wellenlängenmodulationsspektroskopie angepasst ist.

9. Verfahren nach einem der vorhergehenden Ansprüche, **dadurch gekennzeichnet, dass** die Phase der künstlichen Modulation derart angepasst ist, dass bei der Auswertung nach der Wellenlängenmodulationsspektroskopie keine weitere Phasenverschiebung oder Signaldrehung hinzugefügt werden muss um ein optimales Ergebnis zu erhalten.

10. Spektrometer mit

 - einer Lichtquelle zum Erzeugen eines Lichtstrahls mit einer in einem Wellenlängenbereich ([$\lambda$1, $\lambda$2]) variablen Wellenlänge,
 - einem Messvolumen, in dem die zu bestimmende Gaskomponente vorliegt und durch das der Lichtstrahl verläuft,
 - Ansteuermitteln für die Lichtquelle zum Durchstimmen des Wellenlängenbereichs ohne zusätzliche hochfrequente Modulation der Wellenlänge während des Durchstimmens, so dass sich ein monotoner Wellenlänge-Zeit-Verlauf ohne hochfrequente Modulation ergibt,
 - einem Lichtdetektor zum Detektieren der Intensität des Lichtstrahls nach Durchtritt durch das Messvolumen,
 - Speichermitteln zum Speichern von Messpunkten, die jeweils aus einem Zeitpunkt und dazugehörigem Intensitätswert bestehen, während des Durchstimmens, wodurch eine direkte Absorptionslinie erhalten ist,
 - einer Auswerteeinheit zum Erzeugen einer künstlichen Messkurve aus den gespeicherten Messpunkten, durch Verschieben der Messpunkte auf der Zeitachse, wobei die Verschiebung derart erfolgt, dass sich nach Verschiebung in dem Wellenlänge-Zeit-Verlauf eine zusätzliche hochfrequente Modulation ergibt,
 - und zum Auswerten der künstlichen Messkurve nach den Methoden der Wellenlängenmodulationsspektroskopie und Ermitteln eines ersten Konzentrationswertes daraus.

11. Spektrometer nach Anspruch 10, wobei die Auswerteeinheit derart ausgestaltet ist, so dass die Messpunkte, die jeweils aus einem Zeitpunkt und dazugehörigem Intensitätswert bestehen, nach der Methode der direkten Absorptionsspektroskopie ausgewertet werden können und so ein zweiter Konzentrationswert bestimmbar ist.

12. Spektrometer nach Anspruch 10 oder 11, **dadurch gekennzeichnet, dass** die Lichtquelle ein Laser ist, dessen Emissionswellenlänge durch einen Steuerstrom oder eine Steuerspannung veränderbar ist.

13. Spektrometer nach Anspruch 12, **dadurch gekennzeichnet, dass** das Durchstimmen durch lineare Änderung des Steuerstroms oder der Steuerspannung erfolgt.

14. Spektrometer nach Anspruch 12, **dadurch gekennzeichnet, dass** das Durchstimmen durch nicht lineare Änderung des Steuerstroms oder der Steuerspannung erfolgt.

**Claims**

1. A method of determining the concentration of a gas component, comprising:

 - generation of a light beam having a wavelength variable in a wavelength range ([$\lambda$1, $\lambda$2]);
 - guiding the light beam through a measurement volume in which the gas component to be determined is present, wherein the gas component has an absorption in the wavelength range;
 - tuning the wavelength range;
 - detecting the intensity of the light beam after passage through the measurement volume;
 - storage of measurement points that respectively consist of a point in time and of the associated intensity value during the tuning, whereby a direct absorption line is obtained;
 - generating an artificial measurement curve from the stored measurement points by shifting the measurement points on the axis of time;
 - wherein the shift takes place in such a way that an artificial modulation of the wavelength results following the shift in the wavelength time extent; and
 - evaluating the artificial measurement curve in accordance with the methods of wavelength modulation spectroscopy and determining a first concentration value therefrom.

2. A method in accordance with claim 1, **characterized in that** following the storage of the measurement points an evaluation of the direct absorption line is carried out in accordance with the method of the direct absorption spectroscopy and a second concentration value is determined.

3. A method in accordance with claim 2, **characterized in that** the two generated concentration values are used for the purpose of achieving an increased safety with respect to functional safety by a plausibilization of the values with respect to one another.

4. A method in accordance with claim 2 or 3, **characterized in that** a common concentration value is generated from the two concentration values.

**5.** A method in accordance with any one of the preceding claims, **characterized in that** the tuning of the wavelength range takes place without an additional high frequency modulation of the wavelength during the tuning in such a way that a monotonous wavelength time extent results without a high frequency modulation.

**6.** A method in accordance with any one of the preceding claims, **characterized in that** the tuning of the wavelength range in the positive direction is varied with a not necessarily constant velocity >=0 and subsequently a change in the negative direction is varied with a not necessarily constant velocity >=0, in this respect both directions or only one direction can be drawn on for the evaluation.

**7.** A method in accordance with any one of the preceding claims, **characterized in that** the artificial modulation is sinusoidal, rectangular or triangular.

**8.** A method in accordance with any one of the preceding claims, **characterized in that** the amplitude and/or the frequency of the artificial modulation is adapted for the evaluation in accordance with the wavelength modulation spectroscopy.

**9.** A method in accordance with any one of the preceding claims, **characterized in that** the phase of the artificial modulation is adapted in such a way that, on the evaluation in accordance with the wavelength modulation spectroscopy, no further phase shift or signal rotation has to be added in order to achieve an ideal result.

**10.** A spectrometer comprising

- a light source for the generation of a light beam with a wavelength variable in a wavelength range ([λ1, λ2]),
- a measurement volume in which the gas component to be determined is present and through which the light beam propagates;
- control means for the light source for tuning the wavelength range without an additional high frequency modulation of the wavelength during the tuning in such a way that a monotonous wavelength time extent results without high frequency modulation;
- a light detector for detecting the intensity of the light beam after passage through the measurement volume;
- storage means for the storage of measurement points that respectively consist of a point in time and an associated intensity value during the tuning, whereby a direct absorption line is obtained;
- an evaluation unit for generating an artificial measurement curve from the stored measurement points by shifting the measurement points on the axis of time, wherein the shift takes place in such a way that following the shift an additional high frequency modulation results in the wavelength time extent;
- and for evaluating the artificial measurement curve in accordance with the methods of the wavelength modulation spectroscopy and determining a first concentration value therefrom.

**11.** A spectrometer in accordance with claim 10, wherein the evaluation unit is configured in such a way that the measurement points that respectively consist of a point in time and an associated intensity value can be evaluated in accordance with the method of direct absorption spectroscopy and in this way a second concentration value can be determined.

**12.** A spectrometer in accordance with claim 10 or claim 11, **characterized in that** the light source is a laser whose emission wavelength can be changed by a control current or a control voltage.

**13.** A spectrometer in accordance with claim 12, **characterized in that** the tuning takes place by a linear change of the control current or of the control voltage.

**14.** A spectrometer in accordance with claim 12, **characterized in that** the tuning takes place by a nonlinear change of the control current or of the control voltage.

**Revendications**

**1.** Procédé pour la détermination de la concentration d'un composant gazeux, incluant les étapes consistant à :

- engendrer un rayon de lumière avec une longueur d'onde variable dans une plage de longueurs d'onde ([λ1, λ2]),
- guider le rayon de lumière à travers un volume de mesure dans laquelle le composant gazeux à déterminer est présent, le composant gazeux présentant une absorption dans la plage de longueurs d'onde,
- accorder la plage de longueurs d'onde,
- détecter l'intensité du rayon de lumière après sa traversée à travers le volume de mesure,
- pendant la mise en accord, mémoriser des points de mesure, qui sont respectivement constitués d'un instant et d'une valeur d'intensité associée, grâce à quoi on obtient une ligne d'absorption directe,
- engendrer une courbe de mesure artificielle à partir des points de mesure mémorisés, par déplacement des points de mesure sur l'axe des temps,

- dans lequel le déplacement a lieu de telle façon qu'après déplacement il en résulte dans l'évolution longueur d'onde/temps une modulation artificielle des longueurs d'onde,

- évaluation de la courbe de mesure artificielle selon les méthodes de la spectroscopie par modulation des longueurs d'onde et détermination d'une première valeur de concentration à partir de celle-ci.

2. Procédé selon la revendication 1, **caractérisé en ce qu'**après la mémorisation des points de mesure, on exécute une évaluation de la ligne d'absorption directe selon la méthode de spectroscopie par absorption directe, et on détermine une seconde valeur de concentration.

3. Procédé selon la revendication 2, **caractérisé en ce que** les deux valeurs de concentration engendrées sont utilisées, au moyen d'un accord de plausibilité des valeurs l'une par rapport à l'autre, pour atteindre une plus grande sécurité pour ce qui concerne une sécurité fonctionnelle.

4. Procédé selon la revendication 2 ou 3, **caractérisé en ce que** l'on engendre une valeur de concentration commune à partir des deux valeurs de concentration.

5. Procédé selon l'une des revendications précédentes, **caractérisé en ce que** la mise en accord de la plage de longueurs d'onde a lieu sans modulation supplémentaire à haute fréquence des longueurs d'onde pendant la mise en accord, de sorte qu'il en résulte une évolution longueur d'onde/temps monotone sans modulation à haute fréquence.

6. Procédé selon l'une des revendications précédentes, **caractérisé en ce que** la mise en accord de la plage de longueurs d'onde est modifiée en direction positive avec une vitesse >=0 qui n'est pas obligatoirement constante et modification successive en direction négative avec une vitesse >=0 qui n'est pas obligatoirement constante, et ici les deux directions ou seulement une direction peut/peuvent être exploitée(s) pour l'évaluation.

7. Procédé selon l'une des revendications précédentes, **caractérisé en ce que** la modulation artificielle est sous forme sinusoïdale, rectangulaire ou triangulaire.

8. Procédé selon l'une des revendications précédentes, **caractérisé en ce que** l'amplitude et/ou la fréquence de la modulation artificielle est adaptée pour l'exploitation selon la spectroscopie par modulation de longueur d'onde.

9. Procédé selon l'une des revendications précédentes, **caractérisé en ce que** la phase de la modulation artificielle est adaptée de telle façon que lors de l'évaluation selon la spectroscopie par modulation de longueur d'onde, on ne doit ajouter aucun autre décalage de phase ou aucune autre rotation du signal afin d'obtenir un résultat optimal.

10. Spectromètre comprenant

- une source de lumière pour engendrer un rayon de lumière avec une longueur d'onde variable dans une plage de longueurs d'onde ([$\lambda 1$, $\lambda 2$]),
- un volume de mesure dans lequel le composant gazeux à déterminer est présent et à travers lequel se propage le rayon de lumière,
- un moyen de pilotage pour la source de lumière afin d'accorder la plage de longueurs d'onde sans modulation supplémentaire à haute fréquence des longueurs d'onde pendant la mise en accord, de sorte qu'il en résulte une évolution longueur d'onde/temps monotone sans modulation à haute fréquence,
- un détecteur de lumière pour détecter l'intensité du rayon de lumière après avoir traversé le volume de mesure,
- des moyens formant mémoire pour mémoriser des points de mesure qui sont respectivement constitués d'un instant et d'une valeur d'intensité associée, pendant la mise en accord, grâce à quoi on obtient une ligne d'absorption directe,
- une unité d'évaluation pour engendrer une courbe de mesure artificielle à partir des points de mesure mémorisés, par déplacement des points de mesure sur l'axe des temps, dans lequel le déplacement a lieu de telle façon qu'après déplacement dans l'évolution longueur d'onde/temps il en résulte une modulation supplémentaire à haute fréquence,
- et, pour évaluer les points de mesure artificielle selon les méthodes de la spectroscopie par modulation des longueurs d'onde et détermination à partir de celle-ci d'une première valeur de concentration.

11. Spectromètre selon la revendication 10, dans lequel l'unité d'évaluation est conçue de telle façon que les points de mesure, qui sont constitués chacun d'un instant et d'une valeur d'intensité associée, peuvent être évalués selon la méthode de la spectroscopie par absorption directe, et que l'on peut ainsi déterminer une seconde valeur de concentration.

12. Spectromètre selon la revendication 10 ou 11, **caractérisé en ce que** la source de lumière est un laser, dont la longueur d'onde d'émission est modifiable par un courant de commande ou par une tension de commande.

13. Spectromètre selon la revendication 12, **caractérisé en ce que** la mise en accord a lieu par modification linéaire du courant de commande ou de la tension de commande.

14. Spectromètre selon la revendication 12, **caractérisé en ce que** la mise en accord a lieu par modification non linéaire du courant de commande ou de la tension de commande.

10

12

26

20

18

30

28

22

16

14

32

36

Fig. 1

Fig. 2

Wellenlänge λ

Steuerstrom [arb.units]

IA

tp

Zeit

Fig. 3

Intensität I [arb.units]

Ip

A

P

I

λ1    λA    λ2    Wellenlänge λ

Fig. 4

Fig. 5

$P_{WMS}$

Mk

Detektorsignal I [arb.units]

„verschobene" Zeit

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10238356 A1
- US 7616316 B2

- DE 102012223874 B3

**In der Beschreibung aufgeführte Nicht-Patentliteratur**

- Frequency-modulation spectroscopy for trace species detection: theory and comparison among experimental methods. *Applied Optics,* 1992, vol. 31, 707-717

- Background-free absorption spectroscopy using delayed balanced detection. **KRAETSCHMER et al.** Applied Physics B; Lasers And Optics. Springer, 11. Dezember 2009, vol. 98, 249-252